# EUROPEAN PATENT SPECIFICATION

(11) **EP 3 955 987 B1**
(45) Date of publication and mention of the grant of the patent: **12.11.2025**
(21) Application number: 20813959.2
(22) Date of filing: 19.05.2020
(51) Int. Cl.: A61N 1/05, A61F 2/24, A61M 60/191, A61M 60/289, A61M 60/486, A61M 60/515, A61M 60/531, A61M 60/839, A61M 60/861, A61N 1/362, A61M 60/865, A61N 1/378, A61N 1/39

(54) **SMART CARDIAC ASSIST DEVICE**
INTELLIGENTE HERZUNTERSTÜTZUNGSVORRICHTUNG
DISPOSITIF D'ASSISTANCE CARDIAQUE INTELLIGENT

(30) Priority: 28.05.2019 IN 201941021108
(43) Date of publication of application: 23.02.2022
(73) Proprietor: Haval, Sagar S., Coalville Leicestershire LE67 4AP (GB)
(72) Inventor: Haval, Sagar S., Coalville Leicestershire LE67 4AP (GB)
(74) Representative: Cyrson, Matthew Dominic
(86) International application number: PCT/IB2020/054730
(87) International publication number: WO 2020/240344

(56) References cited:
- US-A1- 2004 010 180
- US-A1- 2006 217 774
- US-A1- 2011 021 864
- US-A1- 2014 194 672
- US-A1- 2014 194 717
- US-A1- 2018 116 593
- US-B2- 10 123 875

## Description

### FIELD

The present disclosure herein generally relates to heart compression and assist and arrhythmia control devices. More particularly, the disclosure relates to a smart cardiac assist device.

### BACKGROUND AND PRIOR ART

Congestive Heart failure is a condition in which the heart doesn't pump blood as well as it should. A global study has revealed that India leads in deaths from heart failure. The International Congestive Heart Failure (INTER-CHF) conducted a study aimed at measuring mortality across a year in patients who had suffered a heart attack. The results revealed that 23% Indian heart patients who were part of this study did not survive, whereas only 7% of Chinese succumbed. South East Asian countries showed 15% mortality, while both South America and Middle East had 9% fatalities. Heart failure affects nearly 6 million Americans.

In conditions that over work heart include high blood pressure, valve disease, thyroid disease, kidney disease, diabetes or heart defects present at birth.

In the medical profession, it is recognized that it is necessary, in the event of a cardiac arrest to assist the heart in its pumping action until effective spontaneous cardiac contraction can restore normal blood circulation. During cardiac arrest, the pumping action of the heart ceases and the blood flow stops. If the blood flow or circulation is not re-instituted either by restoration of spontaneous cardiac activity or by some other provision of artificial circulatory support; irreversible vital organ damage and clinical death will ensue.

Such patients with moderate to severe cardiac failure may be less/non - responsive to the conventional medical management. The presently existing Ventricular Assist devices commercially available are more invasive. These devices have lot of complications related to its blood handling properties, unnatural blood flow patterns which affect the end-organs and these devices damage the native heart due to the need to incise heart to install the device. Various cardiac assist devices are known from the prior art.

US 2004/010180 A1 shows a cardiac assist system for treating heart disease and valvular dysfunction, having a contractile transducer to compress the surface of the heart in response to a signal generated by a sensing transducer.

US 2014/194672 A1 shows a heart support system having a constraint sized to fit about at least a portion of an adult human heart in a living body, an expandable chamber disposed within the constraint so as to apply pressure against the heart when expanded, and a connector system including a pneumatic connection port in hydraulic communication with the expandable chamber.

US 2006/217774 A1 shows a cardiac contractile augmentation device (CCAD) comprising an electroactive polymer (EAP) linear segment attached along its length to a selected area of a heart, wherein the EAP segment may be energized during systole to augment the contractile strength of the heart.

US 10 123 875 B2 and US2014/194717 A1 show a device for the support of the cardiac function includes a sheath made of a wire mesh configured to transition from a non-expanded state into an expanded state, with the sheath being self-expanding and being configured to be inserted into a delivery system, and which in the expanded state can at least partially enclose a heart.

Therefore, there is a need for a smart cardiac assist device designed to assist the weak muscles of a failing heart that augments the heart's pumping power and its installation is least invasive. This device could also incorporate pacemaker and de-fibrillator technology to help patients suffering from electrical dysfunctions as bradycardia or tachyarrhythmia.

### OBJECTS

Some of the objects of the present disclosure are described herein below:

The main objective of the present disclosure is to provide a smart cardiac assist device to help increase performance of the heart.

Another objective of the present disclosure is to provide a smart cardiac assist device which helps in leading a near normal function of the diseased heart.

Still another objective of the present disclosure is to provide less invasive approach to treat moderate to severe cardiac failures.

Yet another objective of the present disclosure is to provide more physiological functional assistance to the heart.

Another objective of the present disclosure is to help understand and control heart function in real time.

Another objective of the present disclosure is to provide demand based functional assistance.

Another objective of the present disclosure is to provide pacemaker assistance to the weak heart.

Another objective of the present disclosure is to provide defibrillation assistance to the heart.

Another objective of the present disclosure is to provide data related to blood flow dynamics, cardiac anatomy etc.

The other objectives and advantages of the present disclosure will be apparent from the following description when read in conjunction with the accompanying drawings.

### SUMMARY OF THE INVENTION

The present invention is defined by the appended claims.

Embodiments and examples presented hereinafter are of exemplary nature and are presented to better understand the present invention.

### SUMMARY OF THE PRESENT DISCLOSURE

In view of the foregoing, an embodiment herein provides a smart cardiac assist device which may aid in ventricular recovery. The device proactively assists the left and right ventricular chambers to contract and relax better.

According to an embodiment, the disclosure comprises an implantable and less invasive device to help increase performance of the heart, leading to near normal function of the diseased heart.

According to an embodiment, the device is configured to be installed around the heart in hospital setting, wherein it would form an enclosure to the native patient heart and will assist the left and right ventricular chambers to contract and relax better to help the diseased heart to recover.

According to an embodiment, the device may have capability of self analyzing and suggesting the treatment option for conditions like fibrillation or diminishing electrical signal in the heart. The device may also have a defibrillation and a pacemaker option to treat the heart in case of emergencies.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made but the scope of the present invention is defined by the appended claims.

### BRIEF DESCRIPTION OF DRAWINGS

The detailed description is set forth with reference to the accompanying figures. In the figures, the left-most digit(s) of a reference number identifies the figure in which the reference number first appears. The use of the same reference numbers in different figures indicates similar or identical items.
Fig.1a illustrates one type of structure and basic components of a cardiac assist device, according to an embodiment of the present disclosure herein;
Fig.1b illustrates another type of structure and basic components of a cardiac assist device, according to another embodiment of the present disclosure herein
Fig.2 illustrates structural layers of the device, according to an embodiment of the present disclosure herein;
Fig. 3 illustrates initial packaging of the device and tool for deployment of the device, according to an embodiment of the present disclosure herein;
Fig. 4 illustrates fixation of the device on the heart depicting the location of the sensors, according to an embodiment of the present disclosure here.

### LIST OF NUMERALS

- 100 -: Cardiac assist device
- 101 -: Device
- 102 -: Conduction sensor
- 104 -: Chemical sensor
- 106 -: Pressure sensor
- 108 -: Pacing electrode
- 110 -: Defibrillation electrode
- 112 -: Power and signal conduction
- 114 -: Neutral membrane
- 116 -: Sensor
- 118 -: Actuator
- 120 -: Threaded screw hole
- 121 -: Signal distribution centre
- 122 -: Subcutaneous charging pad
- 123 -: External battery
- 124 -: External processor
- 125 -: Internal battery and internal processor
- 126 -: Memory card
- 127 -: Charger
- 128 -: Smart chip
- 201 -: Mesh layer
- 202 -: Filaments layer
- 203 -: Electrodes and sensors layers
- 204 -: Glue layer
- 301 -: Deployment tool
- 302 -: Screw
- 304 -: Thread
- 306 -: Hand grip
- 401 -: Sensor

### DETAILED DESCRIPTION OF EXEMPLARY EMBODIMENTS:

The embodiments herein and the various features and advantageous details thereof are explained more fully with reference to the non-limiting embodiments and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein.

As mentioned above, there is a need to develop a device for aid during critical conditions to help in ventricular recovering of the heart. The embodiments herein achieve this by providing a smart cardiac assist device to be called as iEnclose, which is configured to help increase performance of the heart, leading to the near normal function of the diseased heart.

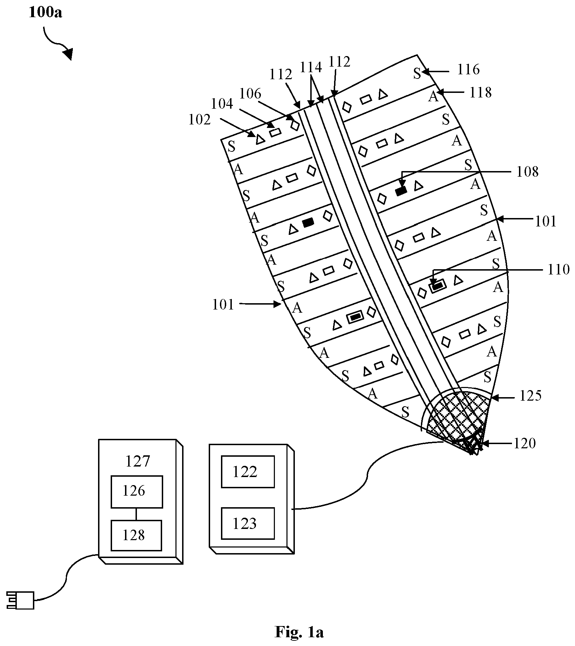

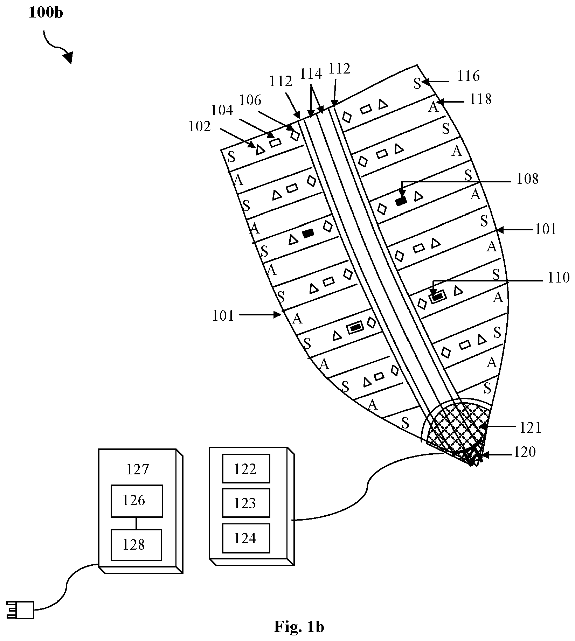

In an embodiment, the device 101 is installed around the heart in a minimal invasive and less complicated way, eliminating the chances of damage to the native heart. Referring now to the drawings, and more particularly to Fig.1 through Fig. 4, where similar reference characters denote corresponding features consistently throughout the figures, there are shown preferred embodiments.

Fig 1a illustrates structure and basic components of a cardiac assist device 101, according to an embodiment of the present disclosure. The cardiac assist device 101 for left ventricle and right ventricle of the heart includes filaments, a plurality of sensors, plurality of actuators, a plurality of electrodes, a threaded hole 120 for a fastener, a power and signal conduction membrane 112, a neutral membrane 114, an internal battery and internal processor 125, a charging pad 122, a memory card 126, a charger 127, and a smart chip 128.

In an embodiment, the plurality of sensors includes conduction sensors 102, chemical sensors 104 and pressure sensors 106. The plurality of sensors are provided for measuring parameters of the heart, wherein the parameters include conduction of electrical impulse, lactate levels, glucose levels, regional saturation of Oxygen (SO2), Regional Carbon di Oxide (CO2) saturation (SCO2), Ischaemic markers like Troponin, Right Atrial Saturations, Left Atrial Saturations, blood pressure from aorta, pulmonary artery, right atrium, left atrium, right ventricle and left ventricle. The conduction sensors 102 measure the conduction of electrical impulse in micro volts. The chemical sensors 104 measure lactate levels, glucose levels, regional saturation of Oxygen (SO2), Regional Carbon di Oxide (CO2) saturation (SCO2), Ischaemic markers like Troponin, Right Atrial Saturations, Left Atrial Saturations. The pressure sensors 106 measure blood pressure from aorta, pulmonary artery, right atrium, left atrium, right ventricle and left ventricle. The measured values from the conduction sensors 102, chemical sensors 104 and pressure sensors 106 are transmitted to the processor.

The filaments are Electro active polymers (EAP) including a plurality of sensors and plurality of actuators. The plurality of electro active polymer (EAP) sensors detects the contraction and relaxation of the heart. The detected signals from the plurality of EAP sensors are transmitted to the plurality of actuators for contracting. There are two types of filaments, contractile filaments and energy generating filaments. Contractile filaments contract and relax in accordance to the cardiac cycle through the electrical signals of the heart. Energy generating filaments contract and relax along with the contractile filaments and generate power and consume energy during the ventricular activity. Energy generating filaments are Electro Active Polymer (EAP) filaments. The Electro Active Polymer (EAP) filaments generate power on stretching, wherein the generated power is stored in capacitors and then transferred to battery. The power stored in the battery will be reused to contract the actuators present in the Electro Active Polymer (EAP) filaments and power the EAP sensors to function accordingly. The filaments can be controlled using various ways. In a first way, controllers are connected physically to the skin for communicating with a subcutaneously placed receiver. The receiver is connected to a processor for transmitting the signal and controlling the filaments. In another way, the filaments can be controlled using a mobile device communicating to the device 101 wirelessly. The filaments can also include a computer connected to the device 101 wirelessly.

The plurality of electrodes includes pacing electrode 108 and defibrillation electrode 110. The pacing electrode 108 and the defibrillation electrode 110 are provided for pacing the heart of a patient. The plurality of electrodes is provided for treating patients suffering from electrical dysfunction such as bradycardia or tachyarrhythmia or conduction defects. The pacing electrode 108 and the defibrillation electrode 110 can be activated automatically or manually based on the need of the cardiac electrical activity. The device 101 also includes ultrasound electrodes for measuring blood flow dynamics and cardiac anatomy.

The device 101 can control the diastolic filling of the heart to prevent over filling and hence over-distension of the heart muscle during cardiac cycle. The Electro Active Polymer sensor filaments of the device 101 are attached to the epicardium for detecting the stretch of the cardiac muscle. On detecting abnormal dilatation of the heart compared to standard dilatation of the heart, the Electro Active Polymer sensor filaments transmit a signal to the processor for activating the actuator.

The power and signal conduction membrane 112 detects a stretch of the the EAP sensor filament which generate power and transmit a signal to the processor to act accordingly. The neutral membrane 114 is present next to the power and signal conduction membrane 112 separating a device 101 of left ventricle from device 101 of right ventricle.

In an embodiment, the device 101 includes an internal battery 125 and internal processor. The battery and processor 125 are inserted in the device 101 placed around the heart. The internal battery 125 powers the device 101 and the internal processor 125 controls the device 101. A subcutaneous charging pad 122 having an external battery 123 is attached to the device 101 through a wire. The subcutaneous charging pad 122 placed away from the device 101, under the skin charges the internal battery 125 of the device 101. The charging pad 122 includes an internal pad. In an embodiment, the charging pad 122 is an induction pad.

In an embodiment, a charger 127 is provided for charging. The charger 127 includes a memory card 126 and a smart chip 128. The smart chip 128 is a SIM card like communication and identification unit, for retrieving data and instructing the device 101 remotely. The smart chip 128 is remotely connected to a cloud, through wireless network. The smart chip 128 receives data from internal pad in the charging pad 122 and transmits data to internal pad in the charging pad 122. The internal pad is a main communication interface between the charging pad 122 used for transferring power to the batteries, calibrating the device 101, and processing commands received from an external controller placed outside the skin. In an embodiment, the data includes measured values of parameters of the heart from the conduction sensors 102, chemical sensors 104 and the pressure sensors 106.

The data received by the smart chip 128 is saved in the memory card 126. The cloud can be accessed through an app provided along with the device 101. In an embodiment, the data from the smart chip of the device 101 can be retrieved in a wired and/or wireless mode. The data can be retrieved intermittently or may be displayed continuously on an external device monitor connected to the cloud. The external or/and internal data storage units may store the data of continuous and intermittent events of the device.

In an embodiment, functions of the device 101 can be controlled in wired, wireless and automatic mode. The device 101 can be controlled wirelessly using software or an app provided with the device 101. In the wired mode the device 101 is controlled from an external computer or mobile device. In the wireless mode the device 101 is controlled from an external mobile device in Short Range Control or Long Range control. In the automatic mode the device 101 automatically understands the ECG signal using the plurality of sensors and functions accordingly.

The device 101 includes internal power storage unit. The internal power storage unit includes battery 125. The internal power storage units can be charged by multiple mechanisms. The multiple mechanisms include recycling unit, internal power generator and external source.

In an embodiment, the recycling unit mechanism consists of energy generated by the energy generating electro active polymer filaments. The recycling unit mechanism may include any other conventional self energy generating unit. In an embodiment, the device 101 produces power from energy generating electro active polymer filaments and the produced power is used for charging the internal battery 125. Hence, the device 101 requires little external power supply.

In an embodiment, the internal power generator mechanism generates power using thermoelectric technology. Thermo-electric generation is harvesting and recovering heat which is converted into electrical energy using thermoelectric generators (TEG).

In an embodiment, the external source mechanism for charging the internal storage unit includes using an induction pad 122 inserted below the skin and an external magnetic induction device. The external source can also include an external backup battery/mains electrical source 127.

In an embodiment, the device 101 is equipped with artificial intelligence for customizing the functions of the device 101 based on a patient's everyday activity and helping in power management.

Fig 1b illustrates structure and basic components of a cardiac assist device 101, according to another embodiment of the present disclosure. 100 b does not include the internal battery 125 and the internal processor 125 based on a feasibility of their placement in the device 101. The battery and processor are provided away from the device 101, subcutaneously. The device 101 includes a signal distribution centre 121 and the device 101 does not include internal battery and internal processor. A subcutaneous charging pad 122 including an external battery 123 and external processor 124 is connected to the device 101 through a wire. The external battery 123 powers the device 101. The subcutaneous processor 124 and battery 123 communicates through a wire with the signal distribution centre 121 in the device 101. The signal distribution centre 121 is a web of wires which distributes signal, power and commands between the processor 124. The processor 124 transfers data to and from the device 101 at low energy.

In an embodiment, the external power storage battery 123 is charged using external power source 127 for recharging the battery and is used as a backup for conditions needing additional power requirements and emergencies.

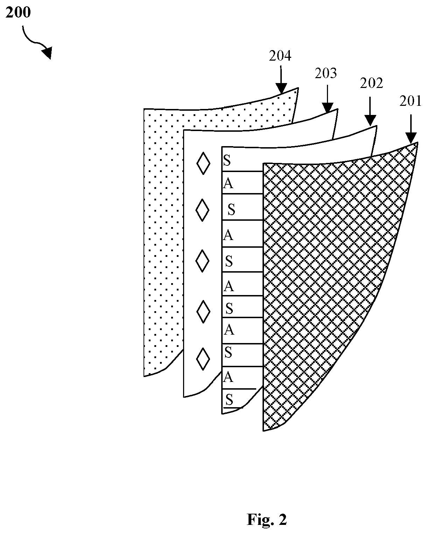

Fig 2 illustrates layers of the smart cardiac assist device, according to an embodiment of the present disclosure. The device 101 includes four layers including a mesh layer 201, a filaments layer 202, an electrode and sensors layer 203 and a glue layer 204.

The mesh layer 201 is made up of a bio-compatible material. In an embodiment, the bio-compatible layer is polyurethane. The bio-compatible materials prevents inflammatory reactions and rejection related issues due to insertion of the device 101 around the heart and helps the device 101 to function normally for longer periods.

The filaments layer 202 includes the electro active polymer (EAP) filaments, the plurality of actuators 118 and the plurality of EAP sensors 116.

The electrode and sensors layer 203 includes the plurality of electrodes and the plurality of sensors. The plurality of electrodes includes pacing electrode 108 and defibrillation electrode 110, the plurality of sensors includes the conduction sensors 102, chemical sensors 104, and the pressure sensors 106.

The glue layer 204 connects and attaches all the layers including the mesh layer 201, the filaments layer 202 and the electrode and sensors layer 203 together.

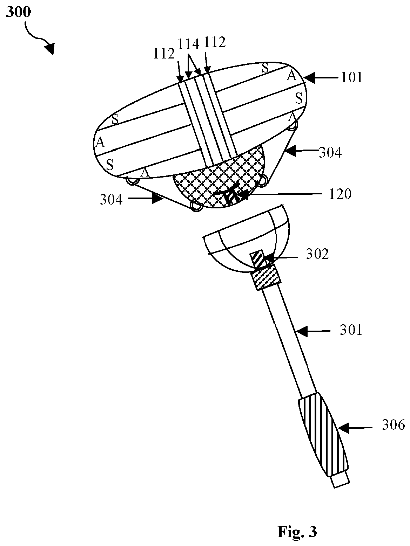

Fig.3 illustrates the initial packaging of the device and tool for deployment of the device, according to an embodiment of the present disclosure. The deployment is performed using a deployment tool 301 having a hand grip 306 and a screw 302.

The device 101 is installed around the heart using the deployment tool 301. The deployment tool includes the screw 302 and the hand grip 306. Material for the hand grip 306 is rubber or metal. The device 101 is folded for inserting using the deployment tool 301. The folded device 101 is held using threads 304 attached to the mesh. The threaded screw hole 120 of the device 101 mates with the screw 302 of the deployment tool 301. The folded device 101 is inserted around the heart through left thoracotomy or midline sternotomy using the deployment tool 301. The incision is made as small as possible, but adequate for visualizing fixation markings and confirming the proper placement of the device 101 on the surface of the heart. Diagnostic tools like ultrasound Echocardiography and or fluoroscopy can be used for helping in positioning the device 101 and checking the proper placement of the device 101 around the heart. The device 101 helps in recovery of the heart, helps in decision making, helps other conventional assist devices, helps in heart transplant. The device 101 can be used as a destination therapy. The device 101 forms an enclosure to the heart of the patient and assists the left and right ventricular chambers of the heart to contract and relax better, helping the diseased heart to recover. In an embodiment, stem cell therapy on heart muscle can be used in conjunction with this device 101 to help regenerate a healthy cardiac tissue in the place of dead/dying tissue.

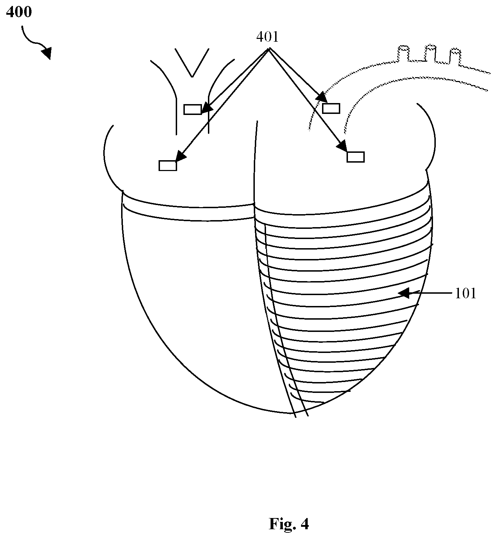

Fig 4 illustrates the fixation of device with location of sensors on heart, according to an embodiment of the present disclosure. The device 101 is installed around the heart and forms an enclosure to the native patient heart. The device 101 can be installed around the left ventricle, right ventricle, or both the left ventricle and right ventricle of the heart.

In an embodiment, the device 101 includes sensors 401 for measuring cardiac muscle strength, native cardiac contraction, blood pressure (from aorta, pulmonary artery, right atrium, left atrium, right ventricle, left ventricle), cardiac electrical signals, regional oxygenation saturation, regional carbon dioxide saturation, regional lactate measurement and cardiac output monitoring (Left Ventricle outflow/aortic flow and right ventricle outflow/pulmonary flow). The sensors 401 are pressure sensors placed on Right Atrium, Pulmonary Artery, Left Atrium, and Aorta.

A main advantage of the present disclosure is that the device provides less invasive approach to treat moderate-severe cardiac failure- causing minimal to no damage to the existing heart muscle, hence providing more time for the impaired cardiac tissue to recover.

Another advantage of the present disclosure is that the device provides more physiological functional assistance by keeping the pulsatile waveforms for better perfusion of body tissues and organs.

Still another advantage of the present disclosure is that the device provides more parameters measured directly from the heart, helping to understand and control heart function in real time.

Yet another advantage of the present disclosure is that the device provides better understanding of the native cardiac function and keeping a track of its recovery/failure.

Another advantage of the present disclosure is that the device provides demand based functional assistance to help the heart to return to its normal function; also it may help provide adequate functional assistance to the heart in various physical activities done by the patient. Hence helping the patient to live near to normal healthy lifestyle.

Another advantage of the present disclosure is that the device has self generating power recycling units helping in less dependency to external power supply and hence prolonging battery life.

Another advantage of the present disclosure is that the device provides wireless retrieval of the device and heart function data.

Another advantage of the present disclosure is that the device has pacemaker and de-fibrillator integrated.

Another advantage of the present disclosure is that the device provides cardiac muscle stretch data and performs regional oxygen and carbon dioxide saturation monitoring of cardiac muscle.

Another advantage of the present disclosure is that the device provides more safety and freedom to the patient due to almost self-dependent control of the device and may send regular updates to the patient and physician on device function and cardiac status.

Another advantage of the present disclosure is that there is possibility of measuring blood flow dynamics and cardiac anatomy directly from the device, whose results may be seen on a wired or wireless device monitor.

Another advantage of the present disclosure is that the device provides wireless remote data visualization or control, using a SIM card like unit.

Another advantage of the present disclosure is that there may be an internal processing unit in the device to learn and adapt to patient physiological conditions.

Another advantage of the present disclosure is that there may be an artificial intelligence driven device function present.

It is to be understood that the phraseology or terminology employed herein is for the purpose of description and not of limitation. Therefore, the embodiments herein can be readily modified or adapted to various applications by applying current knowledge.

The scope of the present invention is however defined by the appended claims.

## Claims

1. A cardiac assist device (101) for a heart, comprising:
a filaments layer (202);
an electrodes and sensors layer (203);
a mesh layer (201) made of bio-compatible material;
a glue layer (204) for connecting the mesh layer (201), the filaments layer (202), and the electrodes and sensors layer (203);
the sensors including conduction sensor (102), chemical sensors (104) and pressure sensors (106) for measuring parameters of the heart;
the mesh layer (201) enclosing the filaments layer (202), the electrodes and sensors layer (203), and the glue layer (204);
the filaments layer (202) including sensors (116) and actuators (118) for contracting and relaxing the heart;
the electrodes including a pacing electrode (108) and a defibrillation electrode (110) for pacing the heart; and
the filaments include contractile filaments for contracting and relaxing the heart and energy generating filaments for generating power.

2. The cardiac assist device as claimed in claim 1, wherein the bio-compatible material of the mesh layer (201) is polyurethane.

3. The cardiac assist device as claimed in claim 1, wherein a deployment tool (301) including a screw (302) for mating with a threaded screw hole (120) of the device is provided for inserting the device (101) around the heart.4.

4. The cardiac assist device as claimed in claim 1, wherein the conduction sensors (102) are configured to measure parameters of conduction of electrical impulse in micro volts, the chemical sensors (104) are configured to measure parameters of lactate levels, glucose levels, regional saturation of Oxygen (SO2), Regional Carbon di Oxide (CO2) saturation (SCO2), Ischaemic markers including Troponin, Right Atrial Saturations, Left Atrial Saturations, the pressure sensors (106) are configured to measure parameters of blood pressure from aorta, pulmonary artery, right atrium, left atrium, right ventricle, left ventricle.

5. The cardiac assist device as claimed in claim 1, wherein the device (101) is is configured to be operated in wired mode, wireless mode and automatic mode.

6. The cardiac assist device as claimed in claim 1, wherein the device (101) includes an internal battery (125) and an internal processor (125) for powering and controlling the device (101).

7. The cardiac assist device as claimed in claim 1, wherein the device (101) includes an external battery (123) and an external processor (124) for powering and controlling the device (101).

8. The cardiac assist device as claimed in claim 1, wherein a battery is configured to be charged using power generated from the energy generating filaments of the device (101).

9. The cardiac assist device as claimed in claim 1, wherein the filaments include electro active polymer (EAP) filaments.

## Patentansprüche

1. Herzunterstützungsvorrichtung (101) für ein Herz, umfassend:
eine Filamentschicht (202);
eine Elektroden- und Sensorschicht (203);
eine Meshschicht (201), die aus einem biokompatiblen Material hergestellt ist;
eine Klebstoffschicht (204) zum Verbinden der Meshschicht (201), der Filamentschicht (202) und der Elektroden- und Sensorschicht (203);
wobei die Sensoren einen Leitungssensor (102), chemische Sensoren (104) und Drucksensoren (106) zum Messen von Parameter des Herzens einschließen;
die Meshschicht (201) die Filamentschicht (202), die Elektroden- und Sensorschicht (203) und die Klebstoffschicht (204) umschließt;
die Filamentschicht (202) Sensoren (116) und Aktuatoren (118) zur Kontraktion und Relaxation des Herzens einschließt;
die Elektroden eine Schrittmacherelektrode (108) und eine Defibrillationselektrode (110) zum Schrittmachen des Herzens einschließen; und
die Filamente kontraktile Filamente zur Kontraktion und Relaxation des Herzens und Energieerzeugungsfilamente zur Erzeugung einer Leistung einschließen.

2. Herzunterstützungsvorrichtung nach Anspruch 1, wobei das biokompatible Material der Meshschicht (201) Polyurethan ist.

3. Herzunterstützungsvorrichtung nach Anspruch 1, wobei ein Einsatzwerkzeug (301), das eine Schraube (302) zum Zusammenpassen mit einem Gewindeschraubenloch (120) der Vorrichtung einschließt, zum Einführen der Vorrichtung (101) um das Herz herum bereitgestellt wird.

4. Herzunterstützungsvorrichtung nach Anspruch 1, wobei die Leitungssensoren (102) dazu konfiguriert sind, Parameter einer Leitung eines elektrischen Impulses in Mikrovolt zu messen, wobei die chemischen Sensoren (104) dazu konfiguriert sind, Parameter von Lactatspiegeln, Glukosespiegeln, einer regionalen Sauerstoffsättigung (SO2), einer regionalen Kohlendioxid-(CO2)-Sättigung (SCO2), ischämischen Markern, einschließlich Troponin, rechtsatrialen Sättigungen, linksatrialen Sättigungen zu messen, wobei die Drucksensoren (106) dazu konfiguriert sind, Parameter eines Blutdrucks von einer Aorta, einer Pulmonalarterie, einem rechten Atrium, einem linken Atrium, einem rechten Ventrikel, einem linken Ventrikel zu messen.

5. Herzunterstützungsvorrichtung nach Anspruch 1, wobei die Vorrichtung (101) dazu konfiguriert ist, in einem drahtgebundenen Modus, einem drahtlosen Modus und einem automatischen Modus betrieben zu werden.

6. Herzunterstützungsvorrichtung nach Anspruch 1, wobei die Vorrichtung (101) eine interne Batterie (125) und einen internen Prozessor (125) zum Antreiben und Steuern der Vorrichtung (101) einschließt.

7. Herzunterstützungsvorrichtung nach Anspruch 1, wobei die Vorrichtung (101) eine externe Batterie (123) und einen externen Prozessor (124) zum Antreiben und Steuern der Vorrichtung (101) einschließt.

8. Herzunterstützungsvorrichtung nach Anspruch 1, wobei eine Batterie dazu konfiguriert ist, unter Verwendung einer Leistung geladen zu werden, die von den Energieerzeugungsfilamenten der Vorrichtung (101) erzeugt wird.

9. Herzunterstützungsvorrichtung nach Anspruch 1, wobei die Filamente elektroaktive Polymerfilamente (EAP-Filamente) einschließen.

## Revendications

1. Un dispositif d'assistance cardiaque (101) pour un cœur, comprenant : une couche de filaments (202) ;
une couche d'électrodes et de capteurs (203) ;
une couche de maille (201) constituée d'un matériau biocompatible ;
une couche de colle (204) pour connecter la couche de maille (201), la couche de filaments (202) et la couche d'électrodes et de capteurs (203) ;
les capteurs comprenant un capteur de conduction (102), des capteurs chimiques (104) et des capteurs de pression (106) pour mesurer les paramètres du cœur ;
la couche de maille (201) entourant la couche de filaments (202), la couche d'électrodes et de capteurs (203) et la couche de colle (204) ;
la couche de filaments (202) comprenant des capteurs (116) et des actionneurs (118) pour contracter et détendre le cœur ;
les électrodes comprenant une électrode de stimulation (108) et une électrode de défibrillation (110) pour stimuler le cœur ; et
les filaments comprennent des filaments contractiles pour contracter et détendre le cœur et des filaments générateurs d'énergie pour générer de l'énergie.

2. Le dispositif d'assistance cardiaque selon la revendication 1, dans lequel le matériau biocompatible de la couche de maille (201) est constitué de polyuréthane.

3. Le dispositif d'assistance cardiaque selon la revendication 1, dans lequel un outil de déploiement (301) comprenant une vis (302) destinée à s'accoupler avec un trou de vis fileté (120) dudit dispositif est prévu pour insérer le dispositif (101) autour du cœur 4.

4. Le dispositif d'assistance cardiaque selon la revendication 1, dans lequel les capteurs de conduction (102) sont configurés pour mesurer des paramètres de conduction d'impulsion électrique en microvolts, les capteurs chimiques (104) sont configurés pour mesurer des paramètres relatifs au niveau de lactate, au niveau de glucose, à la saturation régionale en oxygène (SO2), à la saturation régionale en dioxyde de carbone (CO2), (SCO2), à des marqueurs ischémiques comprenant la troponine, ainsi qu'aux saturations auriculaires droite et gauche ; les capteurs de pression (106) sont configurés pour mesurer des paramètres de pression sanguine au niveau de l'aorte, de l'artère pulmonaire, de l'oreillette droite, de l'oreillette gauche, du ventricule droit et du ventricule gauche.

5. Le dispositif d'assistance cardiaque selon la revendication 1, dans lequel le dispositif (101) est configuré pour fonctionner en mode filaire, en mode sans fil et en mode automatique..

6. Le dispositif d'assistance cardiaque selon la revendication 1, dans lequel le dispositif (101) comprend une batterie interne (125) et un processeur interne (125) pour alimenter et contrôler le dispositif (101).

7. Le dispositif d'assistance cardiaque selon la revendication 1, dans lequel le dispositif (101) comprend une batterie externe (123) et un processeur externe (124) pour alimenter et contrôler le dispositif (101).

8. Le dispositif d'assistance cardiaque selon la revendication 1, dans lequel une batterie est configurée pour être chargée en utilisant l'énergie générée par les filaments générateurs d'énergie du dispositif (101).

9. Le dispositif d'assistance cardiaque selon la revendication 1, dans lequel les filaments comprennent des filaments de polymère électro actif (EAP).
